# EUROPEAN PATENT SPECIFICATION

(11) **EP 0 940 409 B1**
(45) Date of publication and mention of the grant of the patent: **12.05.2004**
(21) Application number: 97930823.6
(22) Date of filing: 17.07.1997
(51) Int. Cl.: C07H 15/04, C07F 9/09, C12P 13/02, C12P 19/44

(54) **PROCESS FOR THE PREPARATION OF SPHINGOLIPIDS AND SPHINGOLIPID DERIVATIVES**
VERFAHREN ZUR HERSTELLUNG VON SPHINGOLIPIDEN UND SPHINGOLIPIDDERIVATEN
PROCEDE DE PREPARATION DE SPHINGOLIPIDES ET DE DERIVES DE SPHINGOLIPIDES

(30) Priority: 19.07.1996 JP 20760696
(43) Date of publication of application: 08.09.1999
(73) Proprietor: TAKARA SHUZO CO. LTD., Kyoto-shi, Kyoto 612 (JP)
(72) Inventor: ITO, Makoto, Fukuoka-shi, Fukuoka 819 (JP); KURITA, Toyohisa, Otsu-shi, Shiga 520-21 (JP); MITSUTAKE, Susumu, Maebaru-shi, Fukuoka 819-11 (JP); KITA, Katsuhiro, Nagasaki-shi, Nagasaki 852 (JP)
(74) Representative: Albrecht, Thomas, Dr.
(86) International application number: PCT/JP1997/002483
(87) International publication number: WO 1998/003529

(56) References cited:
- EP-A- 0 707 063
- WO-A-94/26919
- JP-A- 8 084 587
- JP-T- 7 508 889
- K. DRAUTZ AND H. WALDMANN: ENZYME CATALYSIS IN ORGANIC SYNTHESIS, VCH WEINHEIM, 1995, pages 96-98, XP002151209
- MITSUTAKE, SUSUMU ET AL: "[14C]ceramide synthesis by sphingolipid ceramide N-deacylase: new assay for ceramidase activity detection" ANAL. BIOCHEM. (1997), 247 (1), 52 - 57 , 1997, XP000942968

## Description

### TECHNICAL FIELD

The present invention relates to methods for producing sphingolipids or sphingolipid derivatives which are useful in the fields of medicine, carbohydrate engineering, cell engineering, and the like, and sphingolipids or sphingolipid derivatives obtained by such a production method.

### BACKGROUND ART

"Sphingolipid" is a generic name for lipids having a long-chain base sphingoid such as glycosphingolipids, sphingophospholipids and ceramides, and widely distributed in lower animals to higher animals. Recently, it has been clarified that these sphingolipids participate in important roles in biological activities of cell proliferation, induction of differentiation, apoptosis and the like. Also, attempts have been made to employ these sphingolipids in cosmetics and the like since they are constituents of the cell surface layer.

As a common structure, sphingolipids have a ceramide structure in which a long-chain fatty acid having a nonuniform chain length bonded to the amino group of the sphingoid via an acid-amide bond. With regard to the method for producing sphingolipids or sphingolipid derivatives by modifying or substituting the long-chain fatty acid of sphingolipids, methods are known in which they are synthesized chemically or enzymatically using a lysosphingolipid as the starting material which lacks the fatty acid bonded by the acid-amide bond to the amino group of the sphingoid in the sphingolipid.

As the chemical method, there are methods in which a fatty acid or a fatty acid derivative is condensed to the lyso-form amino group by the following techniques. For example, known are a method in which a fatty acid active ester (for example, *N*-hydroxysuccinimide ester of a fatty acid) is used, a method in which a fatty acid and a coupling agent (for example, carbonyldiimidazole, dicyclohexylcarbodiimide or the like) are used, a method in which a fatty acid anhydride is used, a method in which a fatty acid chloride is used, and the like.

Methods in which a lysoganglioside is used as the lyso-form of an acidic glycolipid are reported in *Methods in Enzymology,* 138:319-341 (1987), European Patent 373039 B1 (1994) and European Patent 765883 A1 (1997). Also, a method in which a sphingosylphosphorylcholine (lysosphingomyelin) is used as the lyso-form of sphingophospholipid is described in *Journal of Lipid Research,* 28:710-718 (1987).

According to these methods, side reactions (for example, *O*-acylation and the like) occur in some cases, so that it is necessary to employ complex steps for use of protecting groups, purification and the like, in order to obtain an *N*-acylated product selectively. Also, when it is required to acylate only the amino group of the sphingoid in a sphingolipid selectively which has an amino group other than the amino group of the sphingoid, such as de-*N*-acetyllysoganglioside which is obtained by chemically deacylating a ceramide ciliatine which is one of sphingophosphonolipids or a glycosphingolipid containing aminosugar, complex steps, such as a step of introduction of protecting groups, partial acylation, partial deacylation after the acylation, and a step of selective *N*-acylation after incorporation of de-*N*-acetyllysoganglioside into liposomes, are required, and therefore it is difficult to conduct the selective acylation.

On the other hand, an enzymatic synthesis method is described in International Publication No. WO 94/26919. In this method, a condensation reaction is carried out by lipase in an organic solvent, so that a substantially water-free organic solvent is required and the substrate is limited depending on its solubility. International Publication No. WO 94/26919 discloses an enzymatic synthesis method of a ceramide and a hybrid ceramide, but the reaction is not specific so that the formation of *O*-acylated products is found. Furthermore, when the substrate has a plurality of amino groups similar to the case of the chemical synthesis methods, it is difficult to carry out the specific reaction with only the amino group of the sphingoid.

As described above, in the previous method for synthesizing sphingolipids or sphingolipid derivatives by chemically or enzymatically modifying or substituting the long-chain fatty acid in the sphingolipid, undesirable by-products are formed, and the substrate is limited. Additionally, in the previous methods, the lysosphingolipid which lacks a fatty acid bonded by an acid-amide bond to the 2-position of the sphingoid in the sphingolipid is used as the starting material. Therefore, when intended sphingolipids:or sphingolipid derivatives are synthesized, it is required to prepare a lysosphingolipid prior to the synthesis.

Accordingly, an object of the present invention is to provide a production method for specifically synthesizing sphingolipids or sphingolipid derivatives having a modified or substituted long-chain fatty acid bonded to the sphingoid from not only a lysosphingolipid but also a sphingolipid.

Also, another object of the present invention is to provide sphingolipids or sphingolipid derivatives obtained by the production method.

### DISCLOSURE OF THE INVENTION

If the present inventions are summarized, the first invention of the present inventions is a method for producing a sphingolipid or a sphingolipid derivative, which comprises enzymatically reacting a sphingolipid with an aliphatic carboxylic acid optionally comprising a marker using a sphingolipid ceramide deacylase (an enzyme which can specifically hydrolyze an acid-amide bond between a sphingoid and a fatty acid in a sphingolipid) to obtain another sphingolipid or sphingolipid derivative having a different fatty acid chain.

The second invention of the present inventions is a method for producing a sphingolipid or a sphingolipid derivative, which comprises enzymatically reacting a lysosphingolipid with an aliphatic carboxylic acid optionally comprising a marker using a sphingolipid ceramide deacylase to obtain a sphingolipid or sphingolipid derivative.

The third invention of the present inventions is a method for producing a sphingolipid or a sphingolipid derivative, which comprises enzymatically reacting at least two different sphingolipids or sphingolipid derivatives using a sphingolipid ceramide deacylase to obtain other sphingolipid or sphingolipid derivative having an exchanged fatty acid chain.

An embodiment of the present inventions is a method for producing a sphingolipid or a sphingolipid derivative, which comprises using a microorganism which is capable of producing the enzyme, instead of the enzyme in the above first to third inventions of the present inventions, and contacting it with the starting material to obtain an objective material.

The present inventors have conducted studies on the synthesis method of a sphingolipid or a sphingolipid derivative, and found as the result that the recombination of a fatty acid to the amino group of the sphingoid of a lysosphingolipid or the substitution of a fatty acid bonded via an acid-amide bond to the sphingoid in a sphingolipid with other fatty acid produces a sphingolipids or sphingolipid derivative by using sphingoid ceramide deacylase and hydrolyzes it into a lysosphingolipid and a fatty acid.

Previously, a reverse reaction or transfer reaction of an enzyme must have been conducted by adding a donor in a large amount excess for an acceptor in an organic solvent system in order to prevent a simultaneously occurring hydrolysis reaction. However, the present inventors have found that a sphingolipid or sphingolipid derivative can be synthesized under mild conditions in an aqueous solution without adding a donor in a large amount excess for an acceptor by using a sphingoid ceramide deacylase, and thereby have accomplished the present invention.

### BRIEF DESCRIPTION OF THE DRAWINGS

Fig. 1 is a graph which shows the specificity of a reverse reaction by an SCDase for fatty acid molecular types.
Fig. 2 is a graph which shows the optimum pH of a reverse reaction by an SCDase.
Fig. 3 is a graph which shows the reaction ratios of a hydrolysis reaction, a reverse reaction and a fatty acid exchange reaction by an SCDase.
Fig. 4 is a graph which compares ceramidase activities in B16 cells.

### BEST MODE FOR CARRYING OUT THE INVENTION

The present invention will be described in detail below.

The term "sphingolipid" as used herein means natural and synthetic substances having long-chain base sphingoid and mixtures thereof, and includes glycosphingolipids, sphingophospholipid and ceramides. The term "lysosphingolipid" as used herein means the *N*-deacylated form of a sphingolipid from which the fatty acid bonded via an acid-amide bond to the amino group of sphingoid has been eliminated.

The "aliphatic carboxylic acid" as used herein involves carboxylic acids having an aliphaticity, such as acids in which a hydrocarbon chain in a fatty acid is substituted with halogen or a functional group (for example, a substituted or unsubstituted amino group, an oxo group, a hydroxyl group or the like), acids having oxygen, sulfur or an amino group in the hydrocarbon chain as well as saturated fatty acids and unsaturated fatty acids.

The term "sphingolipid ceramide deacylase" as used herein means an enzyme which acts on the amide bond of the sphingoid in a sphingolipid, and specifically hydrolyzes the sphingolipid into a lysosphingolipid and a fatty acid, namely, an enzyme which specifically hydrolyzes the acid-amide bond between a sphingoid and a fatty acid in a sphingolipid.

Examples thereof include sphingolipid ceramide deacylases produced by a microorganism belonging to the genus *Pseudomonas* as enzymes which broadly act on a sphingolipid including a glycosphingolipid (ganglioside, neutral glycolipid) and a sphingophospholipid (sphingomyelin) [SCDase, *Journal of Biological Chemistry*, 270:24370-24374 (1995), European Patent 707063 A1 (1996)], ganglioside ceramidases produced by a microorganism belonging to the genus Nocardia as enzymes which act on only ganglioside [*Journal of Biochemistry,* 103:1-4 (1988), U.S. Patent 4,997,760, U.S. Patent 5,143,841], enzymes which are produced by a microorganism belonging to the genus *Rhodococcus* and act on only neutral glycolipid to produce the lyso-form (JP-A-6-78782), glycosphingolipid ceramide deacylases produced by a microorganism belonging to the genus *Streptomyces* as a enzyme which acts on a glycosphingolipid [*Bioscience, Biotechnology, and Biochemistry*, 59:2028-2032 (1995), JP-A-7-107988], and ceramidases which act on a ceramide (acylsphingosine deacylase, EC 3.5.1.23) [*Journal of Biological Chemistry*, 241:3731-3737 (1966), *Biochemistry*, 8:1692-1698 (1969), *Biochimica Biophysica Acta*, 176:339-347 (1969), *Science,* 178:1100-1102 (1972)]. However, the present invention is not limited thereto.

In addition, recombinant enzymes obtained using genes which encode these enzymes and recombinant enzymes obtained using genes which encode these enzymes and are modified by deletion, addition, insertion or substitution are also included in the sphingolipid ceramide deacylase of the present invention, provided that they are enzymes which can specifically hydrolyze an acid-amide bond between a sphingoid and a fatty acid in a sphingolipid.

In using the enzyme, a purified product of the enzyme or a culture broth or crude extract containing the enzyme may be used.

In addition, as described above, a microorganism capable of producing the enzyme may be used instead of the enzyme.

The "microorganism capable of producing a sphingoid ceramide deacylase" as used herein is not restricted thereto so long as the microoranisms are capable of producing a sphingolipid ceramide deacylase. They include microorganisms such as bacteria, yeasts, actinomycetes, hyphomycetes, basidiomycotina, and the like, and cells derived from plants, insects, animals, and the like.

The sphingoid ceramide deacylase or microorganisms capable of producing the enzyme may be immobilized on a well known solid carrier or incorporated into liposome or reversed micelle. An enzyme modified with a high molecular substance may also be used.

The reaction of the present invention progresses in a buffer solution containing sphingolipid or lysosphingolipid to be used as the material, an aliphatic carboxylic acid having or free of a marker and any one of a purified enzyme, a crude extract, a culture broth and a microorganism. In the case of a microorganism, sphingolipid or lysosphingolipid to be used as the material and the aliphatic carboxylic acid may be added to a culture broth of the microorganism. The amount of these materials to be used is not particularly limited, and they can be used to their saturation amount. Generally, it is preferred that the aliphatic carboxylic acid is present in excess amount; however, according to the present invention, the reaction of the sphingolipid or lysosphingolipid with the aliphatic carboxylic acid progresses even at a molar ratio of 1:1, and the sphingolipid or lysosphingolipid may be present in excess amount.

Also, the amount of an enzyme or a microorganism which produces the enzyme is not particularly limited and can be selected at will within a broad range. It may be used, for example, in an approximate amount of 0.1 mU or more, preferably from 3 mU to 10 U, per 1 ml of the starting solution. As the buffer solution, any appropriate buffer solution having a pH value of 5 to 9 may be used, but it is preferred to carry out the reaction in a buffer solution of about pH 6 to 7. Furthermore, it is preferred to add a surfactant to the buffer solution for activating the enzyme or solubilization of the substrate. As the surfactant, a bile acid surfactant, a nonionic surfactant or the like may be used. Although the amount of the surfactant to be added is not particularly limited, it may be used in such an amount that its enzyme activating and substrate solubilizing effects can be obtained or the product can be obtained efficiently, so that it is preferred to add the agent preferably within the range of 0.01% to 2%. Also, an organic solvent may be added to the reaction solution, and when such an organic solvent is used, the reaction may be carried out using a water soluble organic solvent or its two-phase system with an insoluble organic solvent. The amount of the organic solvent to be added is not particularly limited, with the proviso that it is such an amount that the enzyme is not inactivated and the product can be obtained efficiently.

The thus obtained sphingolipid or sphingolipid derivative can be confirmed by thin layer chromatography.

The sphingolipid obtained by the present invention can be isolated and purified by chromatography conventional for organic compounds.

### EXAMPLES

The present invention will be described in detail by the following examples. However, the present invention is not limited thereto.

### EXAMPLE 1

A 50 µl portion of 50 mM acetate buffer (pH 6.0) containing 5 nmol galactosylsphingosine (manufactured by Sigma), 5 nmol [1-¹⁴C] stearic acid (manufactured by Amersham), 0.8% Triton X-100 and 150 µU SCDase derived from the genus *Pseudomonas* [*Journal of Biological Chemistry,* 270:24370-24374 (1995), European Patent 707063 A1 (1996)] was allowed to react overnight at 37°C.

The reaction solution was developed on thin layer chromatography (developing solvent: chloroform/methanol/0.25% magnesium chloride aqueous solution = 65/25/4) and exposed to an imaging plate to obtain a chromatogram by BAS 1000 Imaging Analyzer (manufactured by Fuji Photo Film). In this case, bands of only [1-¹⁴C] stearic acid and a newly formed galactosyl ceramide were detected.

The portion corresponding to the galactosyl ceramide was collected from the thin layer plate and extracted with chloroform/methanol (2/1 by volume). The extract was evaporated to dryness and dissolved in 10 µl of 50 mM acetate buffer (pH 6.0) containing 16 mU β-galactosidase (derived from Jack bean) and 0.4% taurodeoxycholic acid to carry out overnight enzyme digestion at 37°C. The reaction solution was again developed on thin layer chromatography (developing solvent: chloroform/methanol/liquid ammonia = 90/10/1) and analyzed by BAS 1000 Imaging Analyzer (manufactured by Fuji Photo Film) to find a band having the same Rf value of ceramide. Also, when the same reaction, thin layer chromatography and extraction steps were carried out using un-labeled stearic acid and the thus obtained product was analyzed by fast atom bombardment mass spectrometry (FAB-MS), a peak of m/z = 462 which coincided with the parent ion of galactosyl ceramide and a fragment ion peak of m/z = 548 which coincided with the molecular ion peak of ceramide were detected. On the basis of these results, it was revealed that the fatty acid was transferred to the amino group of the sphingosine moiety by a reverse reaction.

### EXAMPLE 2

A 50 µl portion of 50 mM acetate buffer (pH 6.0) containing 50 nmol sphingosylphosphorylcholine (lysosphingomyelin, manufactured by Sigma), 5 nmol [1-¹⁴C] stearic acid, 0.8% Triton X-100 and 150 µU SCDase derived from the genus *Pseudomonas* was allowed to react overnight at 37°C.

The reaction solution was developed on thin layer chromatography (developing solvent: chloroform/methanol/0.02% calcium chloride aqueous solution = 5/4/1) and analyzed by BAS 1000 Imaging Analyzer (manufactured by Fuji Photo Film). In this case, bands of only [1-¹⁴C] stearic acid and a newly formed sphingomyelin were detected.

The portion corresponding to the sphingomyelin was collected from the thin layer plate, extracted and then evaporated to dryness to obtain a reverse reaction product. The product was dissolved in 20 µl of 25 mM phosphate buffer (pH 7.5) containing 35.7 µU sphingomyelinase derived from *Staphylococcus aureus* (manufactured by Sigma) to carry out overnight enzyme digestion at 37°C.

The reaction solution was again developed on thin layer chromatography (developing solvent: chloroform/methanol/liquid ammonia = 90/10/1) and analyzed by BAS 1000 Imaging Analyzer to find a band having the same Rf value of ceramide. On the basis of these results, it was revealed that the fatty acid was transferred to the amino group of the sphingosine moiety by a reverse reaction.

### EXAMPLE 3

### Reverse reaction on various acceptors by SCDase - 1:

A 10 µl portion of 50 mM acetate buffer (pH 6.0) containing 1 nmol [1-¹⁴C] stearic acid, 1 nmol lysosphingolipid, 0.8% Triton X-100 and 30 µU SCDase derived from the genus *Pseudomonas* was subjected to overnight reaction at 37°C. The thus obtained reaction solution was developed on thin layer chromatography and exposed to an imaging plate to carry out for determining the reaction product by BAS 1000 Imaging Analyzer (manufactured by Fuji Photo Film). The results are shown in Table 1.

As shown in Table 1, the enzyme can act not only upon various members of lysoglycosphingolipid broadly but also upon lysosphingophospholipid and sphingosine using them as acceptors.

**TABLE 1**

| Lysosphingolipid | Relative activity (%) |
|---|---|
| Galactosylsphingosine | 100.0 |
| Lysosulfatide | 59.6 |
| Lysolactosyl ceramide | 37.2 |
| Lysogloboside | 34.1 |
| Lysoganglioside GM1a | 15.4 |
| Lysosphingomyelin | 5.7 |
| Sphingosine | 11.5 |

### EXAMPLE 4

### Reverse reaction on various acceptors by SCDase - 2:

A 41.6 µl portion of 25 mM glycine-sodium hydroxide buffer (pH 11) containing 66.6 nmol *N*-trifluoroacetylated aminododecanoic acid, 33.3 nmol lysosphingolipid, 0.3% Triton X-100 and 148 µU SCDase derived from the genus *Pseudomonas* was subjected to 48 hours of reaction at 37°C.

The thus obtained reaction solution was developed on thin layer chromatography, a glycosphingolipid was colored with orcinol-sulfuric acid, and other sphingolipids with Coomassie Brilliant Blue, and then determination of the reaction product was carried out by Imaging Densitometer (manufactured by Bio-Rad). The results are shown in Table 2.

As shown in Table 2, similar to Example 3, the enzyme can act not only on various members of lysosphingolipid broadly but also on sphingosine using it as a receptor.

**TABLE 2**

| Lysosphingolipid | Reaction Efficiency (%) |
|---|---|
| Sphingosine | 69 |
| Lysoganglioside GM3 | 24 |
| Lysoganglioside GD3 | 13 |
| Lysoganglioside GM1a | 29 |
| Lysoganglioside GD1a | 32 |

### EXAMPLE 5

### Specificity of SCDase reverse reaction for fatty acid molecular types:

A 50 µl portion of 50 mM acetate buffer (pH 6.0) containing 5 nmol galactosylsphingosine (manufactured by Sigma), 5 nmol various non-labeled fatty acids, 0.8% Triton X-100 and 150 µU SCDase derived from the genus *Pseudomonas* was allowed to react overnight at 37°C.

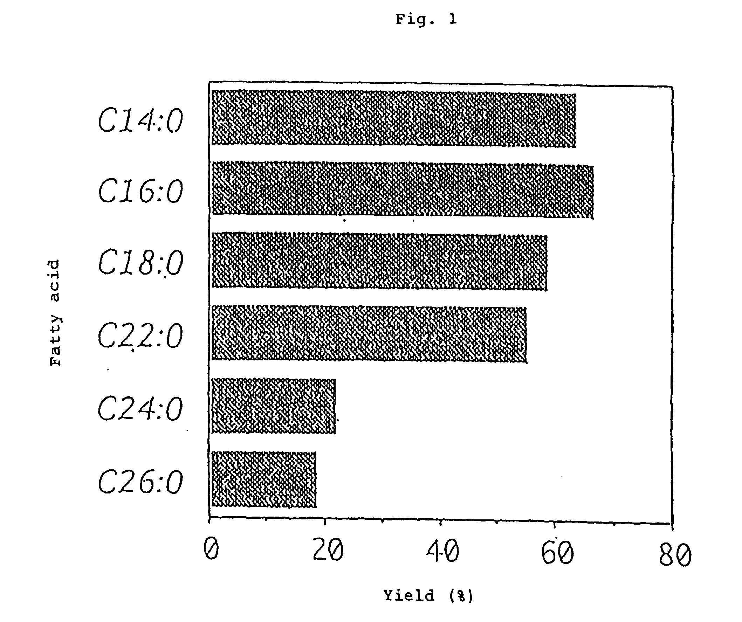

The thus obtained reaction solution was developed on thin layer chromatography, and the reaction products were colored by orcinol-sulfuric acid method and determined using Chromatoscanner CS 9000 (manufactured by Shimadzu Corporation). The results are shown in Fig. 1. That is, Fig. 1 is a graph which shows the specificity of the SCDase reverse reaction for fatty acid molecular types, in which the fatty acid is plotted as ordinate and the yield (%) as abscissa.

### EXAMPLE 6

### Optimum pH of reverse reaction by SCDase:

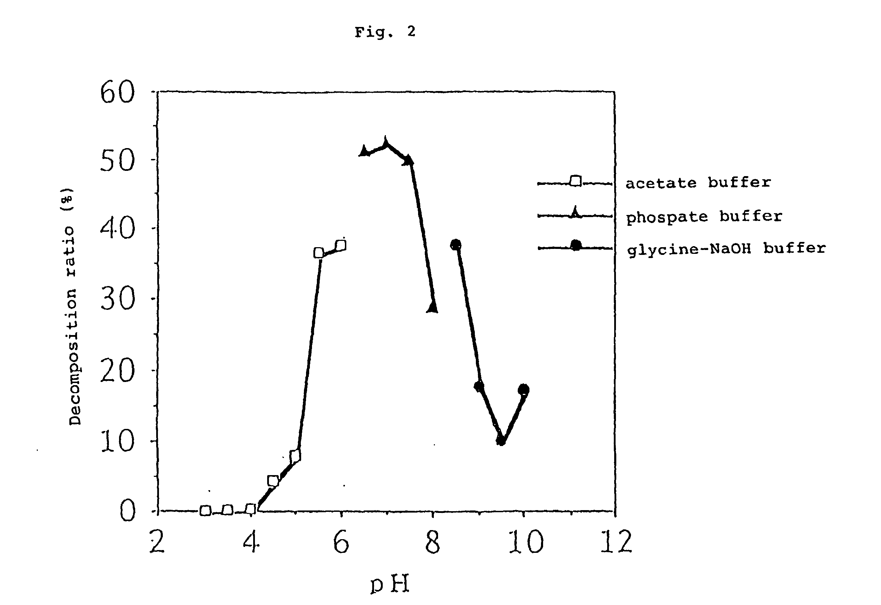

A 10 µl portion of each of various buffer solutions, each containing 1 nmol galactosylsphingosine, 1 nmol [1-¹⁴C] stearic acid, 0.8% Triton X-100 and 30 µU SCDase derived from the genus *Pseudomonas* was allowed to react at 37°C for 3 hours. The results are shown in Fig. 2. That is, Fig. 2 is a graph which shows the optimum pH of the reverse reaction, in which the decomposition ratio (%) is plotted as ordinate and the pH as abscissa. In Fig. 2, □ stands for the acetate buffer, ▲ stands for the phosphate buffer and ● stands for the glycine-NaOH buffer.

### EXAMPLE 7

### Fatty acid exchange reaction on various acceptors by SCDase:

A 10 µl portion of 50 mM acetate buffer (pH 6.0) containing 1 nmol [1-¹⁴C] stearic acid, 1 nmol sphingolipid, 0.8% Triton X-100 and 30 µU SCDase derived from the genus *Pseudomonas* was allowed to react overnight at 37°C.

The thus obtained reaction solution was developed on thin layer chromatography and exposed to an imaging plate to carry out determination of the reaction product by BAS 1000 Imaging Analyzer (manufactured by Fuji Photo Film). The results are shown in Table 3.

As shown in Table 3, the enzyme can perform fatty acid exchange reaction broadly on sphingolipids.

**TABLE 3**

| Sphingolipid | Relative activity (%) |
|---|---|
| Galactosyl ceramide | 100.0 |
| Glucosyl ceramide | 129.0 |
| Sulfatide | 31.7 |
| Lactosyl ceramide | 112.0 |
| Asialo GM1 | 164.0 |
| Globoside | 141.0 |
| Ganglioside GM3 | 54.4 |
| Ganglioside GM2 | 5.5 |
| Ganglioside GM1a | 2.4 |
| Ganglioside GD1a | 6.2 |
| Ganglioside GD1b | 1.2 |
| Sphingomyelin | 2.9 |
| Ceramide | 77.5 |

### EXAMPLE 8

### Examination of reaction conditions:

In order to examine conditions for the hydrolysis reaction, the reverse reaction and the fatty acid exchange reaction, reactions were carried out under the following conditions (A) and (B).

### Reaction conditions (A) :

A 200 µl portion of 25 mM phosphate buffer (pH 6.0) containing 120 µU SCDase derived from the genus *Pseudomonas* and 0.8% Triton X-100 is supplemented, as the substrate, with 100 µM ¹⁴C-galactosyl ceramide at the time of the hydrolysis reaction, 100 µM [1-¹⁴C] stearic acid and 100 µM galactosylsphingosine at the time of the reverse reaction or 100 µM [1-¹⁴C] stearic acid and 100 µM galactosyl ceramide at the time of the fatty acid exchange reaction.

### Reaction conditions (B) :

A 200 µl portion of 25 mM phosphate buffer (pH 7.0) containing 120 µU SCDase derived from the genus *Pseudomonas* and 0.1% Triton X-100 is supplemented, as the substrate, with 100 µM ¹⁴C-galactosyl ceramide at the time of the hydrolysis reaction, 100 µM [1-¹⁴C] stearic acid and 100 µM galactosylsphingosine at the time of the reverse reaction or 100 µM [1-¹⁴C] stearic acid and 100 µM galactosyl ceramide at the time of the fatty acid exchange reaction.

Under the above conditions, each reaction was carried out at 37°C, and a 20 µl portion of the sample was collected from each reaction solution after 0.25, 0.5, 1, 3, 7 or 21 hours of the reaction and heated at 100°C for 5 minutes to stop the reaction.

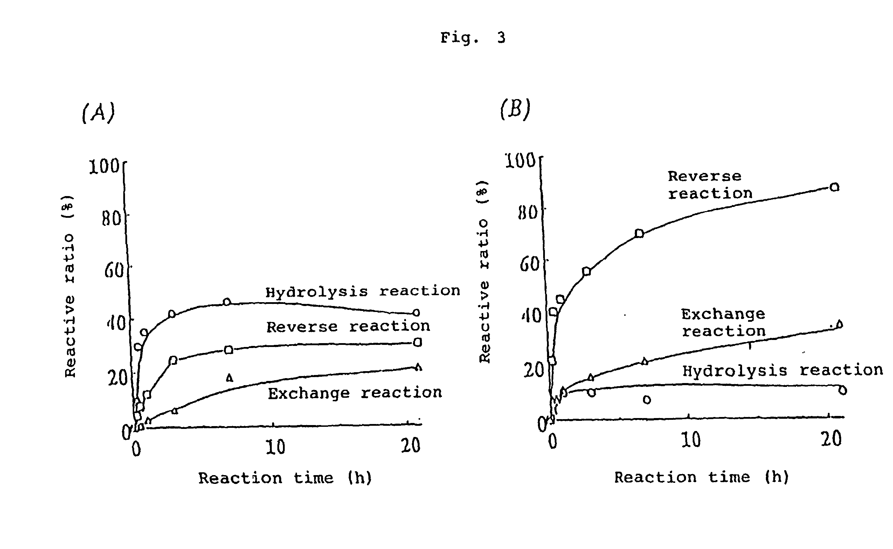

Each of the thus obtained reaction solutions was developed on thin layer chromatography (developing solvent: chloroform/methanol/0.02% calcium chloride aqueous solution = 5/4/1), and the reaction product and unreacted substance were determined by BAS 1000 Imaging Analyzer (manufactured by Fuji Photo Film) to calculate the reaction efficiency. The results are shown in Fig. 3. That is, Fig. 3 is a graph which shows the reaction efficiencies of the hydrolysis reaction, the reverse reaction and the fatty acid exchange reaction by the SCDase under the above-described reaction conditions (A) and (B), in which the reaction ratio (%) is plotted as ordinate and the reaction time (h) as abscissa. In Fig. 3, ○ stands for the hydrolysis reaction, □ stands for the reverse reaction and Δ stands for the fatty acid exchange reaction, each as its reaction ratio.

As the results, it was revealed that the hydrolysis reaction of the SCDase preferentially progresses when the reaction solution has an acidic pH and contains a surfactant in a high concentration, and each of the reverse reaction and fatty acid exchange reaction of the SCDase preferentially progresses when the reaction solution is neutral and when concentration of the surfactant is reduced.

### EXAMPLE 9

### Synthesis of ¹⁴C ceramide:

A 100 nmol (5.0 µCi) portion of [1-¹⁴C] palmitic acid (manufactured by Amersham) and 200 nmol of sphingosine dissolved in ethanol were put into a reaction vessel and completely dried with nitrogen gas. A 0.5 ml portion of 50 mM phosphate buffer (pH 7.0) containing 0.6% Triton X-100 was added to the vessel, thoroughly stirred and then homogenized by ultrasonic treatment. The thus homogenized solution was mixed with 0.5 ml SCDase derived from the genus *Pseudomonas* (1 mU/ml) and was allowed to react at 37°C for 20 hours.

After completion of the reaction, the thus obtained reaction solution was dried using a centrifugation evaporator, the thus dried reaction product was dissolved in 1 ml of hexane/ether/acetic acid (50/50/1 by volume) and applied to Sep-Pak® Silica Cartridge which had been equilibrated with the same solution, unreacted [1-¹⁴C] palmitic acid was washed out with 10 ml of the same solution and then ¹⁴C ceramide was eluted with 10 ml of chloroform/methanol (2/1 by volume).

The eluate was dried with nitrogen gas, suspended in distilled water and then homogenized by ultrasonic treatment. The thus homogenized solution was applied to Sep-Pak® C18 Cartridge. The cartridge was washed with 20 ml of distilled water, and ¹⁴C ceramide was eluted with 3 ml of methanol and 10 ml of chloroform/methanol (2/1 by volume).

Next, the thus obtained eluate was dried with nitrogen gas, dissolved in chloroform/methanol/distilled water (90/10/1 by volume) and then applied to Sep-Pak® CM Cartridge which had been equilibrated with the same solution for adsorbing unreacted sphingosines. In this case, the passed fraction was dried with nitrogen gas to obtain 66 nmol (3.3 µCi) purified ¹⁴C ceramide containing fatty acids and sphingosines at 1% or less.

### EXAMPLE 10

### Synthesis of aminoceramide and its fluorescent derivative:

A 41.6 ml portion of 25 mM glycine-sodium hydroxide buffer (pH 11) containing 66.6 µmol *N*-trifluoroacetylated aminododecanoic acid, 33.3 µmol sphingosine (manufactured by Sigma), 0.3% Triton X-100 and 148 mU *Pseudomonas* SCDase was allowed to react at 37°C for 48 hours.

After completion of the reaction, the reaction solution was applied to C18 reverse phase silica gel column, the column was washed with water for desalting and then *N*-trifluoroacetylated aminoceramide was eluted with chloroform/methanol (2/1 by volume) After evaporation of the solvent, the resulting residue was dissolved in chloroform/methanol/water (90/10/1 by volume) and applied to Sep-Pak® CM Cartridge (manufactured by Waters) for adsorbing unreacted sphingosines and to obtain an unadsorbed fraction containing *N*-trifluoroacetylated aminoceramide. The unadsorbed fraction was applied to Sep-Pak® QMA Cartridge (manufactured by Waters) for adsorbing unreacted *N*-trifluoroacetylated aminododecanoic acid and to obtain an unadsorbed fraction containing *N*-trifluoroacetylated aminoceramide.

A 20 ml portion of chloroform/methanol (2/1 by volume) containing the thus obtained *N*-trifluoroacetylated aminoceramide and 1% sodium methoxide was allowed to react overnight at room temperature. After completion of the reaction, the solvent was evaporated, the resulting residue was suspended in water and applied to Sep-Pak® C18 Cartridge (manufactured by Waters), the column was washed with water to effect desalting and then aminoceramide was eluted with chloroform/methanol (2/1 by volume). After evaporation of the solvent, the resulting residue was dissolved in chloroform/methanol/water (60/30/5), applied to Sep-Pak® CM Cartridge and eluted with chloroform/methanol/1 N HCl (60/30/5), and then the eluent was dried to obtain 5.6 µmol of purified aminoceramide.

A 70 µl portion of 100 nmol aminoceramide dissolved in methanol, 20 µl of 50 mM NBD fluoride (manufactured by Sigma) ethanol solution and 10 µl of triethylamine were allowed to undergo 1 hour of reaction at 60°C. After completion of the reaction, the solvent was evaporated, the resulting residue was dissolved in hexane/ether/acetic acid (50/50/1) and applied to Sep-Pak® Silica Cartridge (manufactured by Waters) and eluted with chloroform/methanol (2/1 by volume), and then the eluate was dried to obtain 30 nmol of purified NBD ceramide.

### EXAMPLE 11

### Screening of Limulus polyphemus ceramidase using fluorescent sphingolipid derivative NBD ceramide:

A 10 µl of serum obtained by centrifugation of Limulus polyphemus blood was allowed to undergo 18 hours of reaction at 37°C with 10 µl of 50 mM acetate buffer (pH 5.0) containing 1 nmol of NBD ceramide prepared in Example 10 and 0.5% Triton X-100. After completion of the reaction, the reaction solution was developed on thin layer chromatography (developing solvent: chloroform/methanol/25% liquid ammonia = 90/20/0.5) and detected under an ultraviolet ray lamp. In this case, a newly formed NBD aminododecanoic acid was detected, so that a ceramidase activity was detected in the Limulus polyphemus serum.

In order to confirm that the ceramidase activity found in the Limulus polyphemus serum is really a ceramidase-derived activity, the ceramidase was purified to find that it was an acidic ceramidase having an optimum pH of 4.5 and a molecular weight of about 205 kDa when measured by a gel filtration method. It was found also that this Limulus polyphemus ceramidase hydrolyzes *N*-stearoylsphingosine (C18:0, d18:1) most efficiently and has activity also on a ceramide containing sphinganine or phytosphingosine as the long-chain base.

Thus, the presence of an invertebrate ceramidase which had not been known was revealed for the first time by the use of the fluorescent sphingolipid derivative NBD ceramide obtained by the production method of the present invention, and it was confirmed that the fluorescent sphingolipid derivative NBD ceramide is useful as a substrate for use in the measurement of ceramidase activity.

### EXAMPLE 12

### Measurement of ceramidase activity in B16 cells using radioisotope labeled ¹⁴C ceramide (C12-¹⁴C-Cer) and fluorescent sphingolipid derivative NBD ceramide (C12-NBD-Cer) as the substrate:

A cell suspension was prepared by suspending 6×10⁶ of B16 cells in 200 µl of 10 mM phosphate buffer. The amount of the protein was determined using MicroBCA™ protein assay reagent (manufactured by Pierce).

### Reaction conditions 1:

Under acidic condition in 10 µl of 50 mM acetate buffer (pH 4.0) containing 10 µl of the cell suspension (diluted to a protein content of 50 µg), 200 pmol of C12-NBD-Cer obtained in Example 10 or 100 pmol of C12-¹⁴C-Cer obtained using lauric acid instead of palmitic acid used in Example 9, as the substrate, and 0.5% Triton X-100.

### Reaction conditions 2:

Under neutral conditions in 10 µl of 50 mM phosphate buffer (pH 7.0) containing 10 µl of the cell suspension (diluted to a protein content of 50 µg), 200 pmol of C12-NBD-Cer or 100 pmol of C12-¹⁴C-Cer as the substrate and 0.5% Triton X-100.

### Reaction conditions 3:

Under basic condition in 10 µl of 50 mM Tris-HCl buffer (pH 8.5) containing 10 µl of the cell suspension (diluted to a protein content of 50 µg), 200 pmol C12-NBD-Cer or 100 pmol C12-¹⁴C-Cer as the substrate and 0.5% Triton X-100.

Under each of the above conditions, the reaction was carried out at 37°C for 3 or 6 hours. Thereafter, the reaction was stopped by adding 100 µl of chloroform/methanol (2/1) to the reaction solution. The thus obtained reaction solution was dried and then dissolved in chloroform/methanol (2/1) to be used as a sample.

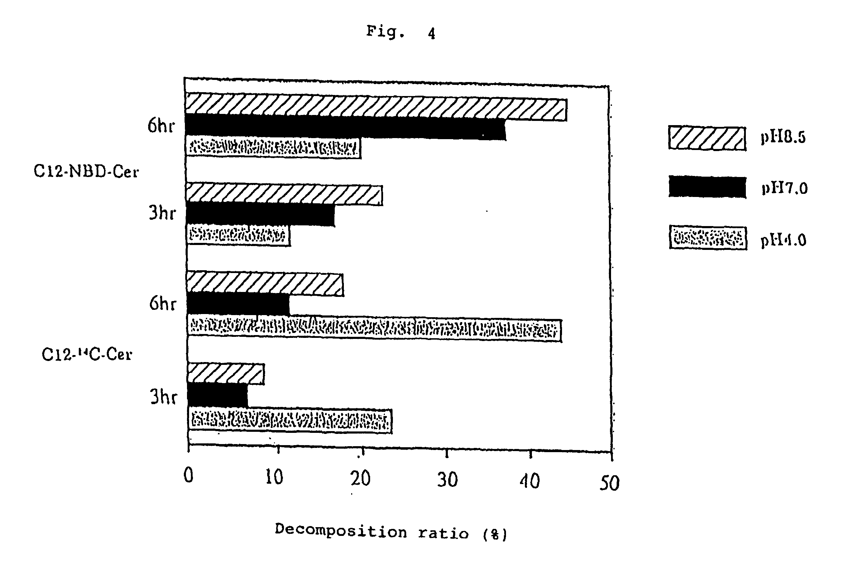

Each of the samples was developed on thin layer chromatography (developing solvent, chloroform/methanol/25% liquid ammonia = 90/20/0.5), and the released ¹⁴C fatty acid was determined using BAS 1000 Imaging Analyzer (manufactured by Fuji Photo Film) to calculate the reaction ratio. Also, the released NBD fatty acid was determined using Chromatoscanner CS 9000 (manufactured by Shimadzu Corporation), and the reaction ratio was calculated. The results are shown in Fig. 4. That is, Fig. 4 is a graph which shows comparison of the measurement of ceramidase activities in B16 cells, in which reactions of 3 hours (3 hr) and 6 hours (6 hr) using C12-NBD-Cer as the substrate and reactions of 3 hours and 6 hours using C12-¹⁴C-Cer as the substrate are plotted in that downward order as ordinate and the decomposition ratio (%) as abscissa.

On the basis of these results, it was suggested that there are an alkaline ceramidase which acts well on C12-NBD-Cer but hardly on C12-¹⁴C-Cer and an acidic ceramidase which acts well on C12-¹⁴C-Cer but hardly on C12-NBD-Cer.

### INDUSTRIAL APPLICABILITY

The present invention provides a method for producing sphingolipid derivatives, which is effected by modifying or substituting a long-chain fatty acid of the ceramide moiety as the common portion of a sphingolipid. Also, the use of the production method renders possible industrially advantageous production of arbitrary sphingolipids or sphingolipid derivatives. Since naturally occurring sphingolipids generally have diversity in terms of the chain length of the long-chain fatty acid, it was difficult to obtain sphingolipids having a uniform chain length of the long-chain fatty acid. However, sphingolipids whose long-chain fatty acid is unified can be obtained by the long-chain fatty acid substitution of the present invention. Also, since it is possible to prepare labeled sphingolipids by introducing a chromophore-forming substance, a fluorescent substance, biotin, a radioactive isotope or the like into the fatty acid moiety of a sphingolipid, the labeled sphingolipids can be applied to the elucidation of intracellular metabolism, transport pathway and the like of sphingolipids. Additionally, by the conversion of the ceramide moiety in the sphingolipid, for example, by the introduction of functional highly unsaturated fatty acid, such as eicosapentaenoic acid (EPA), docosahexaenoic acid (DHA) or the like, new sphingolipid derivatives having modified cell permeability, cell metabolism or biological activity can be created, which can be applied to medicines, cosmetics, cell technology and the like.

The production method of the present invention has rendered possible efficient and low-cost production of arbitrary sphingolipids or sphingolipid derivatives which are useful in medicines, sugar technology, cell technology and the like.

## Claims

1. A method for producing a sphingolipid or a sphingolipid derivative, which comprises the step of enzymatically reacting a sphingolipid with an aliphatic carboxylic acid optionally comprising a marker using a sphingolipid ceramide deacylase to obtain another sphingolipid or sphingolipid derivative having a different fatty acid chain.

2. A method for producing a sphingolipid or a sphingolipid derivative, which comprises the step of enzymatically reacting a lysosphingolipid with an aliphatic carboxylic acid optionally comprising a marker using a sphingolipid ceramide deacylase to obtain a sphingolipid or sphingolipid derivative.

3. A method for producing a sphingolipid or a sphingolipid derivative, which comprises the step of enzymatically reacting at least two different sphingolipids or sphingolipid derivatives using a sphingolipid ceramide deacylase to obtain other sphingolipid or sphingolipid derivative having an exchanged fatty acid chain.

4. The method according to claim 1, which comprises the step of contacting a sphingolipid and an aliphatic carboxylic acid optionally comprising a marker with a microorganism capable of producing a sphingolipid ceramide deacylase to obtain another sphingolipid or sphingolipid derivative having a different fatty acid chain.

5. The method according to claim 2, which comprises the step of contacting a lysosphingolipid and an aliphatic carboxylic acid optionally comprising a marker with a microorganism capable of producing a sphingolipid ceramide deacylase to obtain a sphingolipid or sphingolipid derivative.

6. A method according to claim 3, which comprises the step of contacting at least two different sphingolipids or sphingolipid derivatives with a microorganism capable of producing a sphingolipid ceramide deacylase to obtain other sphingolipid or sphingolipid derivative having an exchanged fatty acid chain.

7. The method according to any one of claims 1-6, wherein the sphingolipid ceramide deacylase is produced by a bacterium belonging to the genus *Pseudomonas*.

8. The method according to claim 4, wherein the microorganism is cultured in a medium containing the sphingolipid and the aliphatic carboxylic acid optionally comprising a marker.

9. The method according to claim 5, wherein the microorganism is cultured in a medium containing the lysosphingolipid and the aliphatic carboxylic acid optionally comprising a marker.

10. The method according to claim 6, wherein the microorganism is cultured in a medium containing the at least two different sphingolipids or sphingolipid derivatives.

11. The method according to any one of claims 4 to 6, wherein the microorganism is a bacterium belonging to the genus *Pseudomonas*.

## Patentansprüche

1. Verfahren zur Herstellung eines Sphingolipids oder eines Sphingolipidderivates, welches den Schritt umfasst, ein Sphingolipid mit einer aliphatischen Carbonsäure, die wahlweise eine Markersubstanz umfasst, unter Verwendung einer Sphingolipid-Ceramid-Deacylase unter Erhalt eines anderen Sphingolipids oder Sphingolipidderivates mit einer anderen Fettsäurekette, enzymatisch umzusetzen.

2. Verfahren zur Herstellung eines Sphingolipids oder eines Sphingolipidderivates, welches den Schritt umfasst, ein Lysosphingolipid mit einer aliphatischen Carbonsäure, die wahlweise eine Markersubstanz umfasst, unter Verwendung einer Sphingolipid-Ceramid-Deacylase unter Erhalt eines Sphingolipids oder eines Sphingolipidderivates, enzymatisch umzusetzen.

3. Verfahren zur Herstellung eines Sphingolipids oder eines Sphingolipidderivates, welches den Schritt umfasst, mindestens zwei unterschiedliche Sphingolipide oder Sphingolipidderivate unter Verwendung einer Sphingolipid-Ceramid-Deacylase unter Erhalt eines anderen Sphingolipids oder Sphingolipidderivates mit einer ausgetauschten Fettsäurekette, enzymatisch umzusetzen.

4. Verfahren nach Anspruch 1, welches den Schritt umfasst, ein Sphingolipid und eine aliphatische Carbonsäure, die wahlweise eine Markersubstanz umfasst, mit einem Mikroorganismus, der in der Lage ist, eine Sphingolipid-Ceramid-Deacylase herzustellen, unter Erhalt eines anderen Sphingolipids oder Sphingolipidderivates mit einer anderen Fettsäurekette, in Kontakt zu bringen.

5. Verfahren nach Anspruch 2, welches den Schritt umfasst, ein Lysosphingolipid und eine aliphatische Carbonsäure, die wahlweise eine Markersubstanz umfasst, mit einem Mikroorganismus, der in der Lage ist, eine Sphingolipid-Ceramid-Deacylase herzustellen, unter Erhalt eines Sphingolipids oder Sphingolipidderivates, in Kontakt zu bringen.

6. Verfahren nach Anspruch 3, welches den Schritt umfasst, mindestens zwei unterschiedliche Sphingolipide oder Sphingolipidderivate mit einem Mikroorganismus, der in der Lage ist, eine Sphingolipid-Ceramid-Deacylase herzustellen, unter Erhalt eines anderen Sphingolipids oder Sphingolipidderivates mit einer ausgetauschten Fettsäurekette, in Kontakt zu bringen.

7. Verfahren nach einem der Ansprüche 1-6, wobei die Sphingolipid-Ceramid-Deacylase von einem Bakterium hergestellt wird, dass zur Gattung *Pseudomonas* gehört.

8. Verfahren nach Anspruch 4, wobei der Mikroorganismus in einem Medium kultiviert wird, das das Sphingolipid und die aliphatische Carbonsäure, die wahlweise eine Markersubstanz umfasst, enthält.

9. Verfahren nach Anspruch 5, wobei der Mikroorganismus in einem Medium kultiviert wird, das das Lysosphingolipid und die aliphatische Carbonsäure, die wahlweise eine Markersubstanz umfasst, enthält.

10. Verfahren nach Anspruch 6, wobei der Mikroorganismus in einem Medium kultiviert wird, das die mindestens zwei unterschiedlichen Sphingolipide oder Sphingolipidderivate enthält.

11. Verfahren nach einem der Ansprüche 4 bis 6, wobei der Mikroorganismus ein Bakterium ist, das zur Gattung *Pseudomonas* gehört.

## Revendications

1. Procédé permettant de produire un sphingolipide ou un dérivé de sphingolipide, qui comprend l'étape consistant à faire réagir enzymatiquement un sphingolipide avec un acide carboxylique aliphatique comprenant éventuellement un marqueur en utilisant une sphingolipide céramide déacylase afin d'obtenir un autre sphingolipide ou dérivé de sphingolipide ayant une chaîne d'acides gras différente.

2. Procédé permettant de produire un sphingolipide ou un dérivé de sphingolipide, qui comprend l'étape consistant à faire réagir enzymatiquement un lysosphingolipide avec un acide carboxylique aliphatique comprenant éventuellement un marqueur en utilisant une sphingolipide céramide déacylase afin d'obtenir un sphingolipide ou un dérivé de sphingolipide.

3. Procédé permettant de produire un sphingolipide ou un dérivé de sphingolipide, qui comprend l'étape consistant à faire réagir enzymatiquement au moins deux sphingolipides ou dérivés de sphingolipides différents en utilisant une sphingolipide céramide déacylase afin d'obtenir un autre sphingolipide ou dérivé de sphingolipide ayant une chaîne d'acide gras échangée.

4. Procédé selon la revendication 1, qui comprend l'étape consistant à mettre en contact un sphingolipide et un acide carboxylique aliphatique comprenant éventuellement un marqueur avec un micro-organisme capable de produire une sphingolipide céramide déacylase afin d'obtenir un autre sphingolipide ou dérivé de sphingolipide ayant une chaîne d'acides gras différente.

5. Procédé selon la revendication 2, qui comprend l'étape consistant à mettre en contact un lysosphingolipide et un acide carboxylique aliphatique comprenant éventuellement un marqueur avec un micro-organisme capable de produire une sphingolipide céramide déacylase afin d'obtenir un sphingolipide ou un dérivé de sphingolipide.

6. Procédé selon la revendication 3, qui comprend l'étape consistant à mettre en contact au moins deux sphingolipides ou dérivés de sphingolipides différents avec un micro-organisme capable de produire une sphingolipide céramide déacylase afin d'obtenir un autre sphingolipide ou un dérivé de sphingolipide ayant une chaîne d'acide gras échangée.

7. Procédé selon l'une quelconque des revendications 1 à 6, dans lequel la sphingolipide céramide déacylase est produite par une bactérie appartenant au genre Pseudomonas.

8. Procédé selon la revendication 4, dans lequel le micro-organisme est cultivé dans un milieu contenant le sphingolipide et l'acide carboxylique aliphatique comprenant éventuellement un marqueur.

9. Procédé selon la revendication 5, dans lequel le micro-organisme est cultivé dans un milieu contenant le lysosphingolipide et l'acide carboxylique aliphatique comprenant éventuellement un marqueur.

10. Procédé selon la revendication 6, dans lequel le micro-organisme est cultivé dans un milieu contenant au moins deux sphingolipides ou dérivés de sphingolipides différents.

11. Procédé selon l'une quelconque des revendications 4 à 6, dans lequel le micro-organisme est une bactérie appartenant au genre Pseudomonas.
